# FASCICULE DE BREVET EUROPEEN

(11) **EP 0 341 112 B1**
(45) Date de publication et mention de la délivrance du brevet: **11.08.1993**
(21) Numéro de dépôt: 89401085.9
(22) Date de dépôt: 19.04.1989
(51) Int. Cl.: C12P 7/44, C12N 1/38

(54) **Procédé de production d'acide itaconique**
Verfahren zur Herstellung von Itaconsäure
Process for the production of itaconic acid

(30) Priorité: 02.05.1988 FR 8805847
(43) Date de publication de la demande: 08.11.1989
(73) Titulaire: RHONE-POULENC CHIMIE, 92408 Courbevoie Cédex (FR)
(72) Inventeur: Cros, Patrick, F-79500 Melle (FR); Schneider, Didier, F-79500 Melle (FR)
(74) Mandataire: Dubruc, Philippe

(56) Documents cités:
- WO-A-87/01725
- WO-A-87/04465
- WO-A-87/06615
- FR-A- 1 293 954
- FR-A- 1 327 937
- CHEMICAL ABSTRACTS, vol. 105, no. 11, 15 septembre 1986, page 513, no. 96031w, Columbus, Ohio, US; N. JU et al.: "Continuous production of glucoamylase and itaconic acid using an immobilized cell bioreactor with intermittent air and liquid contacting", & GONGYE WEISHENGWU 1986, 16(1), 8-16

## Description

La présente invention a pour objet un procédé amélioré pour la production d'acide itaconique par fermentation d'hydrates de carbone au moyen de microorganismes. Elle concerne plus particulièrement un procédé microbiologique par conversion de sucres dérivés de l'amidon.

La synthèse d'acides organiques par fermentation de sucres en présence d'un microorganisme approprié est universellement connue. Des acides typiques de telles fermentations microbiennes comprennent notamment l'acide acétique, l'acide lactique, l'acide citrique, les acides gluconique et 2-cétogluconique, l'acide fumarique, l'acide itaconique. Ces acides sont utiles dans les industries agroalimentaires, pharmaceutiques, chimiques et autres. Des procédé de préparation sont décrits par exemple dans l'ouvrage "Chemicals by fermentation" S.J. Gutcho-Noyes Data Corporation 1973.

Dans les fermentations industrielles, le choix du substrat hydrocarboné est basé à la fois sur sa disponibilité, sur son coût et sur son aptitude à permettre des productivités élevées. L'amidon a souvent été préconisé comme source de carbone bon marché. Cependant, tous les microorganismes ne sont pas capables de métaboliser l'amidon alors que la majorité d'entre eux métabolisent le dextrose. En conséquence, l'amidon doit être hydrolysé/saccharifié avant la fermentation. Il en résulte un coût de production accru.

La présente invention a pour but principal de proposer un procédé de fermentation économique utilisant l'amidon comme source de carbone nutritive et ayant une productivité au moins égale à celle obtenue avec le glucose ou les hydrolysats d'amidon riches en glucose.

Il a été trouvé que l'acide itaconique pouvait être synthétisé en effectuant la fermentation au moyen du microorganisme producteur simultanément à l'hydrolyse enzymatique de l'amidon. Outre le gain de temps résultant de la suppression de l'étape préalable de saccharification de l'amidon, la vitesse de production de l'acide, de manière inattendue, n'est pas affectée comparativement à l'emploi d'un substrat de glucose, même si les conditions de la fermentation (pH et température) sont éloignées des conditions optimales d'activité de l'enzyme hydrolysante.

Selon l'invention, le procédé de production d'acide itaconique par fermentation à l'aide de microorganismes d'un milieu nutritif aqueux contenant de l'amidon comme source de carbone assimilable est caractérisé en ce que la fermentation est conduite en présence additionnelle d'au moins une enzyme amylolytique saccharifiante,

L'amidon utilisé comme source de carbone selon l'invention peut être n'importe quel amidon de céréales comme l'amidon de blé, de maïs, de sorgho, de riz, de tapioca, de seigle, d'avoine ou un amidon de tubercules comme l'amidon de pomme de terre.

L'amidon peut être mis en oeuvre sous forme brute, liquéfiée ou partiellement saccharifiée. Le terme "amidon" inclut selon la présente description l'amidon brut en dispersion aqueuse et les hydrolysats d'amidon résultant de l'hydrolyse incomplète de l'amidon comme l'amidon fluidifié, les sirops d'amidon et les hydrolysats riches en dextrose. Les hydrolysats d'amidon diffèrent entre eux par leur degré d'hydrolyse, exprimé en équivalent dextrose D.E. et leur contenu en oligosaccharides et polysaccharides plus élevés. Les amidons fluidifiés présentent un D.E. compris entre environ 3 et 20 et renferment généralement 50 à 95 % de polysaccharides, de degré de polymérisation supérieur à G 7 (7 unités glucose). Les sirops d'amidon ou sirops de glucose de faible équivalent dextrose présentent un D.E. allant de environ 20 à 68 avec 10 à 50 % de polysaccharides supérieurs à G 7. Les hydrolysats d'amidon ou sirops riches en dextrose ont un D.E. pouvant atteindre 90-98 %. La préparation des hydrolysats d'amidon est bien connu dans la technique. Conventionnellement, les amidons fluidifiés et les sirops d'amidon sont obtenus par hydrolyse acide et/ou enzymatique au moyen d'une α amylase de liquéfaction, éventuellement suivi d'une β amylase. Pour les hydrolysats riches en glucose, l'amidon est le plus souvent transformé selon un procédé en 2 étapes, par action d'une α amylase de liquéfaction puis par action d'une enzyme de saccharification comme la glucoamylase, connue aussi sous le nom d'amyloglucosidase.

Dans la pratique du procédé selon l'invention on utilisera de préférence un sirop d'amidon et encore plus préférentiellement un amidon fluidifié. L'emploi des hydrolysats riches en glucose n'est pas intéressant du point de vue économique étant donné qu'il implique une saccharification préalable qui est une étape longue, même dans les conditions d'activité maximales de l'amyloglucosidase qui se situent généralement à une température supérieure à 50°C et à pH de 4,5-5.

L'amidon et ses produits d'hydrolyse peuvent être employés directement dans le procédé de l'invention sous forme non purifiée, après stérilisation. On peut bien entendu mettre en oeuvre des produits commerciaux purifiés, concentrés ou déshydratés comme les maltodextrines.

L'amidon est présent dans le milieu de fermentation en quantité nécessaire pour fournir 1 à 15 % en poids de glucose par rapport au milieu de fermentation. Exprimé en amidon brut, sur une base sèche, des quantités convenables peuvent être comprises entre 10 et 160 g/l, de préférence entre 60 et 140 g/l du milieu de fermentation.

Les enzymes amylolytiques de saccharification que l'on ajoute selon le procédé de l'invention dans le milieu de fermentation contenant le microorganisme producteur d'acide itaconique sont capables de transformer les dextrines de l'amidon en glucose et maltose. Comme enzymes de saccharification on peut citer les α amylases saccharifiantes comme l'α amylase de Bacillus subtilis var.amylosaccharitiens, l'α amylase fongique, les β amylases, la glucoamylase, l'isoamylase, la pullulanase. Ces enzymes peuvent être employées seules ou en mélange entre elles.

On donne la préférence à la glucoamylase du fait de sa haute spécificité. Le glucoamylase peut être toute glucoamylase fongique telle que celles appartenant aux genres Aspergillus, Endomyces ou Rhizopus. Dans le cas notamment où l'on utilise l'amidon brut comme source de carbone, il est encore possible d'employer un enzyme liquéfiante en addition à l'enzyme saccharifiante, par exemple un mélange α amylase liquéfiante - β amylase ou α amylase liquéfiante - glucoamylase. Des préparations enzymatiques industrielles sont décrites dans l'ouvrage Encycl. of Pol. Sc. Vol 6, pp 46-53 (1967).

L'enzyme amylolytique de saccharification, éventuellement de liquéfaction, est ajoutée dans le milieu de fermentation en quantité nécessaire pour effectuer la saccharification, respectivement la fluidification, de l'amidon. La quantité minimale utile est fonction de l'activité de l'enzyme, de la quantité et du D.E. de l'amidon présent dans le milieu et peut être déterminée facilement par l'homme de métier. De manière générale, on utilise des quantités suffisantes pour fournir 0,04 à 2 unités d'activité enzymatique, de préférence 0,1 à 1 unité, par gramme d'amidon (exprimé en matières sèches). A titre d'exemple d'enzyme saccharifiante, l'AMG 200 L®, commercialisée par NOVO INDUSTRY et qui est une glucoamylase, peut être ajoutée en quantité de 0,02 à 1 %, de préférence entre 0,05 et 0,5 % en poids basé sur le poids de solides contenu dans l'amidon liquéfié présent dans le milieu de fermentation.

N'importe quel microorganisme susceptible de produire l'acide itaconique en présence de sucres peut être utilisé dans le procédé de l'invention. On peut notamment citer les champignons appartenant aux espèces Aspergillus itaconicus et Aspergillus terreus, la préférence étant donnée à ce dernier.

En dehors de la source de carbone et de l'enzyme amylolytique utilisées selon l'invention, le milieu de production et les conditions de la fermentation peuvent être choisis parmi ceux décrits dans la littérature.

La source d'azote peut notamment être choisie parmi les composés organiques et/ou minéraux métabolisables, tels que l'extrait soluble de maïs (CSL) et/ou de soja, l'urée, le sulfate d'ammonium, chlorure d'ammonium, phosphate d'ammonium, nitrate d'ammonium, etc... et leurs mélanges. Le milieu contient en outre des sels minéraux tels que sulfates, chlorures, phosphates de Ca, Mg, Na, K, Fe, Ni, Co, Cu, Mn, Zn, ainsi que d'autres additifs conventionnels tels que des agents de contrôle de pH et/ou des agents anti-moussants.

Le microorganisme est introduit dans le milieu de fermentation de manière connue en soi à l'aide d'inoculums ou de cultures d'intermédiaires.

L'enzyme amylolytique est, de préférence, ajoutée dans le milieu stérile immédiatement avant inoculation. La fermentation est convenablement réalisée à un pH acide pouvant aller de environ 1,8 à 5 et à une température d'environ 20°C à environ 40°C, les conditions optimales dépendant de la souche particulière du microorganisme employé. Des pourcentages élevés d'acide itaconique ont été obtenus en utilisant Aspergillus terreus à un pH préférentiellement compris entre 2 et 4, et plus préférentiellement entre 2 et 3,4, la température étant maintenue à environ 30-40°C.

Après achèvement de la fermentation, l'acide itaconique produit peut être récupéré du moût et purifié selon les méthodes connues telles que filtration, concentration, cristallisation ou extraction par solvants.

On comprendra que la description ci-dessus vise un procédé spécifique pour préparer de l'acide itaconique par fermentation à l'aide de microorganismes d'un milieu contenant une source d'hydrate de carbone produite à partir d'amidon, en présence d'enzymes capables d'hydrolyser l'amidon ou ses produits de dégradation en mono- et disaccharides et que l'invention elle-même ne se limite pas à la composition précise du milieu de fermentation ni aux modes de réalisation particuliers.

Les exemples suivants illustrent l'invention.

### EXEMPLE 1

### Préparation d'amidon brut fluidifié

Une solution aqueuse d'amidon de blé à 350 g/l de matière sèche est homogénéisée, ajustée à pH 6,5 et additionnée de 0,175 g/l d'une enzyme de liquéfaction (TERMANYL 120L® - Novo Industry). La solution est alimentée dans un stérilisateur à injection de vapeur. La température est maintenue à 100-105°C pendant 7 minutes, refroidie à 95°C et conservée à cette température pendant 2 heures dans un bac agité, puis refroidie à 35°C.

### Fermentation

La solution d'amidon fluidifié contenant 130 kg de matières sèches est introduite dans un fermenteur de 1400 litres. On ajoute
- une solution nutritive stérilisée 30 mn à 100°C contenant 0,5 kg d'extrait de maïs, 3,45 kg de chlorure de magnésium, 0,3 kg de sulfate de magnésium, 0,9 kg d'urée, 0,4 kg de chlorure de sodium, 0,033 kg de sulfate de zinc, 0,05 kg de phosphate monopotassique, 1 kg de chlorure de calcium, 0,06 kg de sulfate de cuivre et 0,3 kg d'acide sulfurique. Le pH est de 3,6.
- 0,29 kg d'amyloglucosidase (AMG 200L® - Novo Industry).

Ce milieu de production dont le volume final est ajusté à 1000 litres est agité, aéré et ensemencé par 20 litres d'une culture d'Aspergillus terreus NRRL 1960 de 35 heures d'âge réalisée à 32-35°C dans un fermenteur contenant 25 g/l de glucose, 4,5 g/l de sulfate de magnésium, 0,4 g/l de chlorure de sodium, 0,004 g/l de sulfate de zinc, 0,1 g/l de phosphate monopotassique, 0,5 g/l d'extrait de maïs, 2,0 g/l de nitrate d'ammonium et 0,5 g/l d'acide sulfurique.

La température du milieu est régulée à 32-35°C.

La fermentation est arrêtée après épuisement du sucre et lorsque l'acidité est maxima et stable.

Des échantillons de moût sont prélevés périodiquement pour évaluation de la teneur en acide itaconique déterminée par chromatographie liquide à haute performance.

| Durée de fermentation (heures) | Acide itaconique accumulé (g/l) |
|---|---|
| 18 | 4,55 |
| 24 | 13 |
| 43 | 41,6 |
| 75 | 70,3 |

La productivité est de 0,937 g/l.h.

### EXEMPLE 2 (comparatif)

On réalise une fermentation de manière identique à celle de l'exemple 1 à l'aide de la même culture d'Aspergillus Terreus. Le milieu de production est identique, sauf, que l'on n'ajoute pas de glucoamylase et que l'amidon liquéfié est remplacé par la même quantité (exprimée en matières sèches) d'un hydrolysat d'amidon obtenu préalablement par saccharification enzymatique selon le processus suivant :
Après liquéfaction de l'amidon réalisée de la manière indiquée à l'exemple 1, la solution est refroidie à 60°C, ajustée à pH 4,5, additionnée de 168,4 g d'amyloglucosidase AMG 200L®, et maintenue à 60°C pendant 60 heures. La solution (DE ≧ 96) est refroidie à 30-35°C et introduite dans le fermenteur.

Après addition de la solution nutritive, le milieu est ensemencé et la fermentation conduite dans les conditions de l'exemple 1. La fermentation est arrêtée après épuisement du glucose et lorsque l'acidité est maxima et stable.

L'analyse des échantillons prélevés périodiquement donne les résultats suivants :

| Durée de fermentation (heures) | Acide itaconique accumulé (g/l) |
|---|---|
| 18 | 0,65 |
| 43 | 26,65 |
| 66 | 61,7 |
| 85 | 74,35 |

La productivité est de 0,874 g/l.h.

### EXEMPLE 3 (comparatif)

Une fermentation est réalisée de manière identique à celle de l'exemple 1, mais en l'absence d'amyloglucosidase et en remplaçant l'amidon fluidifié par 150 kg de sirop de glucose purifié ayant un contenu en matières sèches de 74 % et un DE de 96-98 commercialisé par la Société ROQUETTE.

L'analyse des échantillons prélevés au cours de la fermentation donne les résultats suivants :

| Durée de fermentation (heures) | Acide itaconique accumulé (g/l) |
|---|---|
| 23 | 3,25 |
| 41 | 18,2 |
| 64 | 43,5 |
| 94 | 64,35 |

La productivité est de 0,684 g/l.h.

### EXEMPLE 4

### Essai A (comparatif)

Dans un fermenteur aéré et agité, on introduit 36 litres d'une solution d'amidon liquéfié DE 10-15 préparée à partir d'une solution à 180 g/l d'amidon de blé qui est homogénéisée, additionnée de 4,86 g d'enzyme Termamyl 120L® ajustée à pH 6,5, chauffée à 102°C pendant 1 heure puis refroidie à 35°C.

On ajoute une solution nutritive stérilisée 30 mn à 100°C contenant 25g d'extrait de maïs, 172 g de chlorure de magnésium, 15 g de sulfate de magnésium 7H₂O, 45 g d'urée, 20 g de chlorure de sodium, 1,7 g de sulfate de zinc, 2,5 g de phosphate monopotassique, 50 g de chlorure de calcium, 3 g de sulfate de cuivre et 15 g d'acide sulfurique.

Le milieu ayant un volume final de 50 l et pH 3,6 est ajusté à 32-35°C, et ensemencé par 1 l d'une culture d'Aspergillus Terreus NRRL 1960 de 35 heures d'âge réalisée comme décrit dans l'exemple 1.

La fermentation est réalisée à 32-35°C, avec une agitation de 200 t/mn et une vitesse d'aération de 2 m³/h, le pH variant librement durant la fermentation.

La durée de la fermentation est de 112 heures. Le moût contient 47,5 g/l d'acide itaconique, soit une productivité de 0,424 g/l.h.

### Essai B

On opère comme dans l'essai A, mais avant inoculation on ajoute dans le milieu de production 18 g d'amyloglucosidase AMG 200L®.

La durée de fermentation réalisée dans les conditions de l'essai A est de 80 heures. Le moût contient 74,1 g/l d'acide itaconique, soit une productivité de 0,926 g/l.h.

### EXEMPLES 5 A 9

On réalise une série de fermentations comme décrit dans l'exemple 4 en faisant varier la quantité d'amyloglucosidase additionnée dans le milieu avant inoculation par la culture d'Aspergillus Terreus.

Les résultats figurent dans le Tableau suivant :

| | Exemples | | | | |
|---|---|---|---|---|---|
| | 5 | 6 | 7 | 8 | 9 |
| Amidon liquifié g/l (en matières sèches) | 130 | 130 | 130 | 130 | 130 |
| AMG 200L® g/l | 0,16 | 0,29 | 0,36 | 0,42 | 0,65 |
| Durée fermentation heures | 90 | 83 | 80 | 78 | 70 |
| Acide produit g/l | 67,3 | 70,9 | 74,1 | 73,3 | 69,6 |
| Rendement/équivalent glucose % | 53,3 | 56,2 | 58,7 | 58,1 | 55,3 |
| Productivité g/l.h. | 0,747 | 0,854 | 0,926 | 0,94 | 0,99 |

## Revendications

1. Procédé de production d'acide itaconique par fermentation, au moyen de microorganismes, d'un milieu nutrifif aqueux contenant de l'amidon comme source de carbone assimilable caractérisé en ce que la fermentation est conduite en présence additionnelle d'au moins une enzyme amylolytique saccharifiante.

2. Procédé selon la revendication 1 caractérisé en ce que l'amidon est mis en oeuvre sous forme liquéfiée ou partiellement saccharifiée.

3. Procédé selon la revendication 1 caractérisé en ce que l'amidon est mis en oeuvre sous forme brute.

4. Procédé selon la revendication 3 caractérisé en ce que le milieu de fermentation contient une enzyme de liquéfaction en addition à l'enzyme saccharifiante.

5. Procédé selon l'une des revendications 1 à 4 caractérisé en ce que l'amidon est présent en quantité nécessaire pour fournir 1 à 15 % en poids de glucose par rapport au milieu de fermentation.

6. Procédé selon l'une des revendications 1 à 5 caractérisé en ce que l'enzyme saccharifiante est choisie parmi les α amylases, les β amylases, la glucoamylase, l'isoamylase, la pullulanase et leurs mélanges.

7. Procédé selon l'une des revendications 1 à 6 caractérisé en ce que l'enzyme saccharifiante est utilisée en quantité suffisante pour fournir 0,04 à 2 unités d'activité enzymatique, de préférence 0,1 à 1 unité, par g d'amidon exprimé en matières sèches.

8. Procédé selon l'une des revendications 1 à 7 caractérisé en ce que l'enzyme saccharifiante est une glucoalymase et est utilisée en quantité comprise entre 0,02 et 1 %, de préférence entre 0,05 et 0,5 %, en poids basé sur le poids de solides contenu dans l'amidon.

9. Procédé selon l'une des revendications 1 à 8 caractérisé en ce que le microorganisme est choisi parmi les champignons appartenant aux espèces Aspergillus itaconicus et Aspergillus terreus.

## Claims

1. A process for the production of itaconic acid by fermentation, by means of micro-organisms, of an aqueous nutrient medium containing starch as an assimilable carbon source characterised in that fermentation is carried out in the additional presence of at least one saccharifying amylolytic enzyme.

2. A process according to claim 1 characterized in that the starch is used in liquefied or partially saccharified form.

3. A process according to claim 1 characterized in that the starch is used in raw form.

4. A process according to claim 3 characterised in that the fermentation medium contains a liquefaction enzyme in addition to the saccharifying enzyme.

5. A process according to one of claims 1 to 4 characterised in that the starch is present in an amount necessary to provide from 1 to 15% by weight of glucose with respect to the fermentation medium.

6. A process according to one of claims 1 to 5 characterised in that the saccharifying enzyme is selected from α amylases, β amylases, glucoamylase, isoamylase, pullulanase and mixtures thereof.

7. A process according to one of claims 1 to 6 characterised in that the saccharifying enzyme is used in a sufficient amount to provide from 0.04 to 2 units of enzymatic activity, preferably from 0.1 to 1 unit, per g of starch expressed as dry matter.

8. A process according to one of claims 1 to 7 characterised in that the saccharifying enzyme is a glucoamylase and is used in an amount of between 0.02 and 1%, preferably between 0.05 and 0.5%, by weight, based on the weight of solids contained in the starch.

9. A process according to one of claims 1 to 8 characterised in that the micro-organism is selected from fungi belonging to the species Aspergillus itaconicus and Aspergillus terreus.

## Patentansprüche

1. Verfahren zur Herstellung von Itaconsäure durch Fermentation eines wäßrigen Nährmediums, das Stärke als Quelle für assimilierbaren Kohlenstoff enthält, mittels Mikroorganismen, dadurch gekennzeichnet, daß die Fermentation in Gegenwart von zusätzlich mindestens einem stärkespaltenden verzuckernden Enzym durchgeführt wird.

2. Verfahren nach Anspruch 1, dadurch gekennzeichnet, daß die Stärke in verflüssigter oder teilweise verzuckerter Form eingesetzt wird.

3. Verfahren nach Anspruch 1, dadurch gekennzeichnet, daß die Stärke roh eingesetzt wird.

4. Verfahren nach Anspruch 3, dadurch gekennzeichnet, daß das Fermentationsmedium ein Verflüssigungsenzym zusätzlich zum verzuckernden Enzym enthält.

5. Verfahren nach einem der Ansprüche 1 bis 4, dadurch gekennzeichnet, daß die Stärke in der notwendigen Menge vorhanden ist, um 1 bis 15 Gew.-% Glucose, bezogen auf das Fermentationsmedium, zu liefern.

6. Verfahren nach einem der Ansprüche 1 bis 5, dadurch gekennzeichnet, daß das verzuckernde Enzym unter den α-Amylasen, β-Amylasen, Glucoamylase, Isoamylase, Pullulanase und deren Gemischen ausgewählt wird.

7. Verfahren nach einem der Ansprüche 1 bis 6, dadurch gekennzeichnet, daß das verzuckernde Enzym in ausreichender Menge eingesetzt wird, um 0,04 bis 2 Einheiten enzymatischer Aktivität, vorzugsweise 0,1 bis 1 Einheit je g Stärke, ausgedrückt als Trockensubstanz, zu liefern.

8. Verfahren nach einem der Ansprüche 1 bis 7, dadurch gekennzeichnet, daß das verzuckernde Enzym eine Glucoamylase ist und in einer Menge von 0,02 bis 1 Gew.-%, vorzugsweise von 0,05 bis 0,5 Gew.-%, bezogen auf die in der Stärke enthaltenen Feststoffe, eingesetzt wird.

9. Verfahren nach einem der Ansprüche 1 bis 8, dadurch gekennzeichnet, daß der Mikroorganismus unter den Pilzen ausgewählt wird, die zu den Spezies Aspergillus itaconicus und Aspergillus terreus gehören.
